# EUROPEAN PATENT APPLICATION

(11) **EP 3 508 124 A1**
(43) Date of publication of application: **10.07.2019**
(21) Application number: 17846751.0
(22) Date of filing: 04.09.2017
(51) Int. Cl.: A61B 5/1455, A61B 10/00

(54) **BIOLOGICAL MEASUREMENT DEVICE AND BIOLOGICAL MEASUREMENT METHOD**

(30) Priority: 05.09.2016 JP 2016172933
(71) Applicant: Hamamatsu Photonics K.K., Hamamatsu-shi, Shizuoka 435-8558 (JP); National University Corporation Hamamatsu University School of Medicine, Hamamatsu-shi, Shizuoka 431-3192 (JP)
(72) Inventor: YOSHIMOTO Kenji, Hamamatsu-shi Shizuoka 435-8558 (JP); UEDA Yukio, Hamamatsu-shi Shizuoka 435-8558 (JP); OHMAE Etsuko, Hamamatsu-shi Shizuoka 435-8558 (JP); MIMURA Tetsuya, Hamamatsu-shi Shizuoka 435-8558 (JP); YOSHIZAWA Nobuko, Hamamatsu-shi Shizuoka 431-3192 (JP); NASU Hatsuko, Hamamatsu-shi Shizuoka 431-3192 (JP); OGURA Hiroyuki, Hamamatsu-shi Shizuoka 431-3192 (JP); SAKAHARA Harumi, Hamamatsu-shi Shizuoka 431-3192 (JP)
(74) Representative: Hoffmann Eitle
(86) International application number: PCT/JP2017/031829
(87) International publication number: WO 2018/043748

(57) **Abstract**

A biological measurement device includes a probe including a contact surface that includes a plurality of light input portions that input light to a measurement target part and a plurality of light receiving portions that receive measurement light output from the measurement target part, the contact surface being pressed against the measurement target part, and a calculation unit that calculates internal information on the basis of a result of detecting the measurement light obtained for each combination of the light input portion and the light receiving portion, wherein the plurality of light input portions and the plurality of light receiving portions constitute lattices having an interval d in a first direction and a second direction orthogonal to each other on the contact surface, at least some of the plurality of light receiving portions are located at a position separated by a distance A×d (A is an integer equal to or greater than 2) in the first direction and the second direction from the light input portion, at least some of the plurality of light input portions are located at a position separated by the distance A×d in the first direction and the second direction from the light receiving portion, and the combination is a combination in which an interval between the light input portion and the light receiving portion is the distance A×d.

## Description

### Technical Field

An aspect of the present invention relates to a biological measurement device and a biological measurement method.

### Background Art

Patent Literature 1 describes a technology regarding a biological optical measurement device. In the device described in this Literature, a plurality of light irradiators that irradiate an examination target body with light and a plurality of light receivers that receive the light radiated from the light irradiator and propagating through the examination target body are alternately arranged on the examination target body. The device sets a substantial middle point position of the light irradiator and the light receiver as a measurement point and measures a concentration of a metabolite inside the examination target body and a change in the concentration on the basis of a signal detected by the light receiver.

### Citation List

### Patent Literature

Patent Literature 1: Japanese Unexamined Patent Publication No. 2001-178708
Patent Literature 2: Japanese Unexamined Patent Publication No. 2005-049238

### Summary of Invention

### Technical Problem

A device using so-called diffuse optical tomography (DOT) that obtains internal information using light absorption characteristics of a living body has been proposed as a device that noninvasively measures internal information of a living body such as a head or a breast. In such a measurement device, a part of a living body that is a measurement target can be irradiated with light from a predetermined irradiation position, light propagating while being scattered inside the part can be detected at a predetermined detection position, and internal information of the part, that is, information (a position, a concentration, or the like) on a light absorber such as a tumor or hemoglobin present inside the part can be obtained from a result of measurement of an intensity, a time waveform, or the like of the light.

In such a measurement device, a scheme of performing irradiation of a measurement target part with light and detection of diffused light using a compact probe that can be gripped is conceivable. That is, this scheme is a scheme using, for example, diffused reflected light among light diffused inside the measurement target part for measurement. With such a scheme, it is possible to realize a simpler measurement device that is easier to use. However, in this scheme, since the irradiation with the light and the detection of the diffused light are performed in a limited narrow region on a surface of the measurement target part, there is a problem that uniformity of measurement sensitivity easily becomes degraded.

An aspect of the present invention has been made in view of such problems, and an object of the present invention is to provide a probe-type biological measurement device and a biological measurement method that can improve uniformity of measurement sensitivity.

### Solution to Problem

A biological measurement device according to an aspect of the present invention is a biological measurement device that inputs light to a measurement target part of a subject and acquires internal information of the measurement target part by detecting measurement light propagating through the measurement target part, the biological measurement device including: a probe including a contact surface that includes a plurality of light input portions that input the light to the measurement target part and a plurality of light receiving portions that receive the measurement light output from the measurement target part, the contact surface being pressed against the measurement target part; and a calculation unit that calculates the internal information on the basis of a result of detecting the measurement light obtained for each combination of the light input portion and the light receiving portion. The plurality of light input portions and the plurality of light receiving portions constitute lattices having an interval d, and along a first direction and a second direction orthogonal to each other on the contact surface, at least some of the plurality of light receiving portions are located at a position separated by a distance A×d (A is an integer equal to or greater than 2) in the first direction and the second direction from the light input portion, and at least some of the plurality of light input portions are located at a position separated by the distance A×d in the first direction and the second direction from the light receiving portion. The combination is a combination in which an interval between the light input portion and the light receiving portion is the distance A×d.

Further, a biological measurement method according to an aspect of the present invention is a biological measurement method for inputting light to a measurement target part of a subject and acquiring internal information of the measurement target part by detecting measurement light propagating through the measurement target part, the biological measurement method including a step of disposing a probe including a contact surface that includes a plurality of light input portions and a plurality of light receiving portions at the measurement target part (a disposition step); a step of inputting the light from one of the plurality of light input portions to the measurement target part (a light input step); a step of detecting the measurement light received by the light receiving portion located at a position separated by a distance A×d (A is an integer equal to or greater than 2) from the light input portion that has input the light among the plurality of light receiving portions when an interval between lattices configured of the plurality of light input portions and the plurality of light receiving portions on the contact surface is d, and outputting a detection result (a detection step); and a step of calculating the internal information on the basis of the detection result (a calculation step).

According to this biological measurement device and biological measurement method, since the internal information is calculated by a combination in which the distance between the light input portion and the light receiving portion is the distance A×d, a density of the combinations of the light input portions and the light receiving portions is uniform, and uniformity of measurement sensitivity can be improved.

The plurality of light input portions may sequentially input the light to the measurement target part. In this case, S/N can be improved.

The integer A may be 2. In this case, a large number of combinations can be obtained.

The lattice interval d may be equal to or greater than 0.5 cm and less than or equal to 1 cm. In this case, it is possible to improve resolution while suppressing saturation at the time of light detection.

The calculation unit (the calculation step) may create a two-dimensional image or a three-dimensional image on the basis of the internal information. Since a density of the combinations of the light input portions and the light receiving portions is uniform, it is possible to create a clear image.

### Advantageous Effects of Invention

According to the aspect of the present invention, it is possible to provide a probe-type biological measurement device and a biological measurement method that can improve uniformity of measurement sensitivity.

### Brief Description of Drawings

FIG. 1 is a diagram conceptually illustrating a configuration of an embodiment of a biological measurement device.
FIG. 2 is a plan view schematically illustrating an arrangement of a light irradiation portion and a light receiving portion.
FIGS. 3(a) and 3(b) are diagrams illustrating basic patterns of an arrangement of light irradiation portions and light receiving portions.
FIG. 4 is a diagram illustrating combinations of light irradiation portions and light receiving portions.
FIG. 5 is a plan view schematically illustrating an example of an arrangement of light irradiation portions and light receiving portions.
FIG. 6 is a plan view schematically illustrating an example of an arrangement of light irradiation portions and light receiving portions.
FIG. 7 is a plan view schematically illustrating an example of an arrangement of light irradiation portions and light receiving portions.
FIG. 8 is a plan view schematically illustrating an example of an arrangement of light irradiation portions and light receiving portions.
FIG. 9 is a plan view schematically illustrating an example of an arrangement of light irradiation portions and light receiving portions.
FIG. 10 is a plan view schematically illustrating an example of an arrangement of light irradiation portions and light receiving portions.
FIG. 11 is a plan view schematically illustrating an example of an arrangement of light irradiation portions and light receiving portions.
FIG. 12 is a flowchart illustrating a biological measurement method according to an embodiment.
FIG. 13 is a front view illustrating left and right breasts of a breast cancer patient including a measurement target part.
FIGS. 14(a) and 14(b) illustrate a result of measuring the measurement target part illustrated in FIG. 13 and obtaining a CT image.
FIGS. 15(a) and 15(b) illustrate a two-dimensional mapping image obtained by measuring the measurement target part illustrated in FIG. 13.
FIGS. 16(a) to 16(c) are diagrams illustrating a process of an embodiment.
FIG. 17 is a diagram illustrating combinations of light irradiation portions and light receiving portions in a comparative example.
FIGS. 18(a) and 18(b) are diagrams illustrating combinations of light irradiation portions and light receiving portions in the comparative example.
FIGS. 19(a) and 19(b) are diagrams illustrating combinations of the light irradiation portions and the light receiving portions in the comparative example.
FIG. 20 is a diagram illustrating a position and a shape of an absorber inside a solid phantom, CT images indicating a three-dimensional distribution of an absorption coefficient inside the solid phantom acquired in a comparative example, and CT images indicating a three-dimensional distribution of an absorption coefficient inside a solid phantom acquired in the example.
FIG. 21 is a diagram illustrating a position and a shape of an absorber inside a solid phantom, CT images indicating a three-dimensional distribution of an absorption coefficient inside the solid phantom acquired in a comparative example, and CT images indicating a three-dimensional distribution of an absorption coefficient inside a solid phantom acquired in the example.
FIG. 22 is a diagram illustrating a position and a shape of an absorber inside a solid phantom, CT images indicating a three-dimensional distribution of an absorption coefficient inside the solid phantom acquired in a comparative example, and CT images indicating a three-dimensional distribution of an absorption coefficient inside a solid phantom acquired in the example.
FIGS. 23(a) and 23(b) are diagrams illustrating an arrangement of light irradiators and light receivers described in Patent Literature 1, combinations of the light irradiators and the light receivers, and measurement points in each combination.

### Description of Embodiments

Hereinafter, embodiments of a biological measurement device and a biological measurement method will be described in detail with reference to the accompanying drawings. It should be noted that in the description of the drawings, the same elements are denoted by the same reference numerals, and repeated description thereof is omitted.

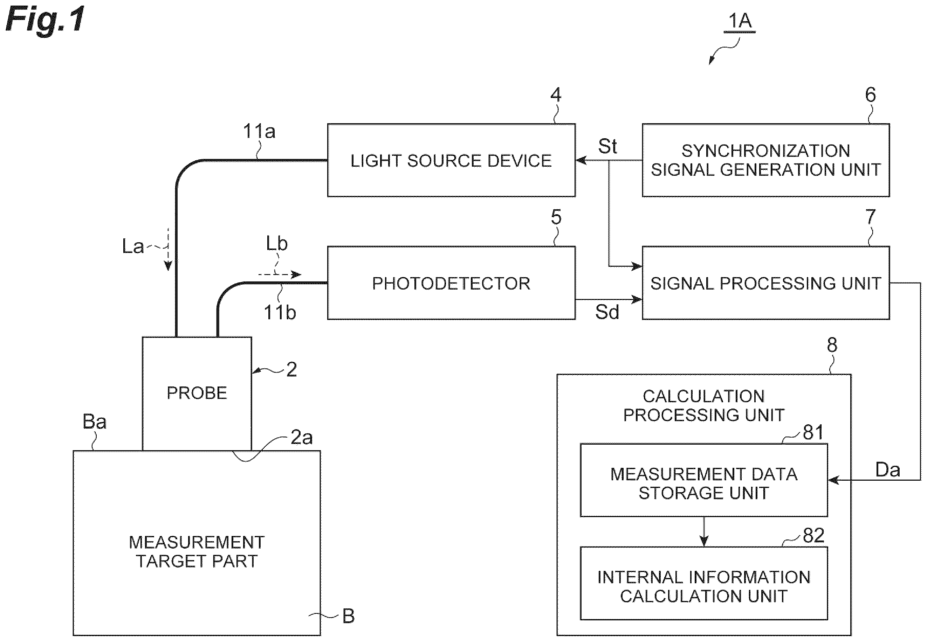

FIG. 1 is a diagram conceptually illustrating a configuration of an embodiment of a biological measurement device and a biological measurement method. A biological measurement device 1A according to the present embodiment is a device for irradiating a measurement target part (for example, a head or a breast) B of a subject with light La which is near-infrared light, detecting diffused reflected light (measurement light) Lb propagating through the measurement target part to acquire internal information (an optical coefficient such as a scattering coefficient and an absorption coefficient, concentrations of various light absorption substances (for example, a tumor or blood hemoglobin), and the like) of the measurement target part B, converting the internal information into an image, and examining for the presence or absence of a tumor, cancer, or the like. It should be noted that the measurement light is not limited to the diffused reflected light.

As illustrated in FIG. 1, the biological measurement device 1A includes a probe 2 that is arranged on a surface of the measurement target part B. The probe 2 has a substantially rectangular parallelepiped shape having a predetermined direction as a longitudinal direction, and a width of the probe 2 in a lateral direction is a size that can be gripped with one hand. The probe 2 includes a contact surface 2a which is pressed against the measurement target part B at one end in a longitudinal direction. Further, the probe 2 holds one end of each of a plurality of irradiation optical fibers 11a for radiating light and a plurality of detection optical fibers 11b for detecting diffused reflected light. It should be noted that one optical fiber 11a and one optical fiber 11b are illustrated as representative in FIG. 1. Respective end surfaces of the optical fibers 11a and 11b are fixed toward a surface Ba of the measurement target part B in a predetermined arrangement (layout) on the contact surface 2a.

Further, the biological measurement device 1A includes light source devices 4, photodetectors 5, a synchronization signal generation unit 6, a signal processing unit 7, and a calculation processing unit 8. The plurality of light source devices 4 are provided to correspond to the number of irradiation optical fibers 11a, and generate light La that is radiated to the measurement target part B. As the light source devices 4, various light sources such as light emitting diodes (LEDs) and light sources such as laser diodes (LDs) can be used. Further, an aspect of the light La output from the light source devices 4 is determined according to a method of calculating internal information in the calculation processing unit 8. For example, various types of light such as pulse light and continuous light (CW light) are adopted. As the pulsed light, pulsed light having a short time width is used so that internal information of the measurement target part B can be measured. For example, a time width in a range of 1 nanosecond or less is selected. The light source devices 4 are optically coupled to the contact surface 2a of the probe 2 via the irradiation optical fiber 11a. The irradiation optical fibers 11a guide the light La emitted from the light source devices 4 to a predetermined irradiation position on the contact surface 2a of the probe 2. The light La is radiated toward the surface Ba of the measurement target part B from the output end. It should be noted that input of the light La to the measurement target part B is not limited to radiation of the light La toward the surface Ba of the measurement target part B.

The light La radiated toward the measurement target part B propagates inside the measurement target part B to become diffused reflected light Lb. The diffused reflected light Lb is emitted from the surface Ba of the measurement target part B and output from the measurement target part B. The detection optical fibers 11b receive the diffused reflected light Lb emitted from the surface Ba of the measurement target part B at a predetermined light receiving position and guide the diffused reflected light Lb to the photodetectors 5.

The plurality of photodetectors 5 are provided to correspond to the number of detection optical fibers 11b. The photodetectors 5 are optically coupled to the contact surface 2a of the probe 2 via the detection optical fibers 11b and detect an intensity of the diffused reflected light Lb obtained from the measurement target part B via the detection optical fibers 11b. The photodetectors 5 output a light detection signal Sd indicating the intensity of the diffused reflected light Lb. The light detection signal Sd output from the photodetectors 5 is input to the signal processing unit 7. As the photodetectors 5, various photodetectors such as photomultiplier tubes, photodiodes, avalanche photodiodes, PIN photodiodes, and single photon avalanche diodes (SPADs) can be used. It should be noted that the photodetectors 5 can have spectral sensitivity characteristics capable of sufficiently detecting a wavelength band of the light La output from the light source devices 4. Further, it is possible to use the photodetectors 5 with high sensitivity or a high gain when the diffused reflected light Lb from the measurement target part B is weak.

The synchronization signal generation unit 6 outputs a trigger signal St for causing the light source devices 4 and the signal processing unit 7 to operate in synchronization with each other (for matching an irradiation timing of the light La at each irradiation position with a detection timing of the diffused reflected light Lb at a corresponding light receiving position). Each of the light source devices 4 described above outputs the light La in synchronization with the timing indicated by the trigger signal St.

The signal processing unit 7 is electrically connected to each of the photodetectors 5 and the synchronization signal generation unit 6, and receives the light detection signal Sd output from each photodetector 5 and the trigger signal St output from the synchronization signal generation unit 6. The signal processing unit 7 acquires the light detection signal Sd of each light receiving position corresponding to each irradiation position in synchronization with the timing indicated by the trigger signal St for each light source device 4. The signal processing unit 7 generates measurement data Da indicating a temporal change (measured waveform) in the light intensity of the diffused reflected light Lb obtained from the light receiving position corresponding to each irradiation position. The signal processing unit 7 provides the acquired measurement data Da to the calculation processing unit 8.

The calculation processing unit (a calculation unit) 8 is realized by a computer including a calculation means such as a central processing unit (CPU), and a storage means such as a memory. Examples of such a computer include a personal computer, a smart device such as a smartphone or a tablet terminal, and a cloud server. The calculation processing unit 8 is electrically connected to the signal processing unit 7, analyzes the measurement data Da input from the signal processing unit 7 to calculate internal information of the measurement target part B, and performs conversion of the internal information into an image (image reconstruction). The calculation processing unit 8 includes a measurement data storage unit 81 and an internal information calculation unit 82. The measurement data storage unit 81 stores the measurement data Da input from the signal processing unit 7 to the calculation processing unit 8. Measurement data for reference that is used for calculation of the internal information may also be stored in the measurement data storage unit 81. The internal information calculation unit 82 analyzes the measurement data Da to calculate the internal information of the measurement target part B and converts the internal information into an image. The calculation of the internal information is performed by applying analysis calculation using, for example, time resolved spectroscopy (TRS) using a time resolved waveform of the diffused reflected light Lb, phase modulation spectroscopy (PMS) using modulated light as the light La, or a method using continuous wave (CW) light as the light La. As a detailed method of calculating the internal information, for example, a method described in Patent Literature 2 can be used. It should be noted that the calculation processing unit 8 may further include a function of controlling at least one of components such as the light source devices 4, the photodetectors 5, and the signal processing unit 7.

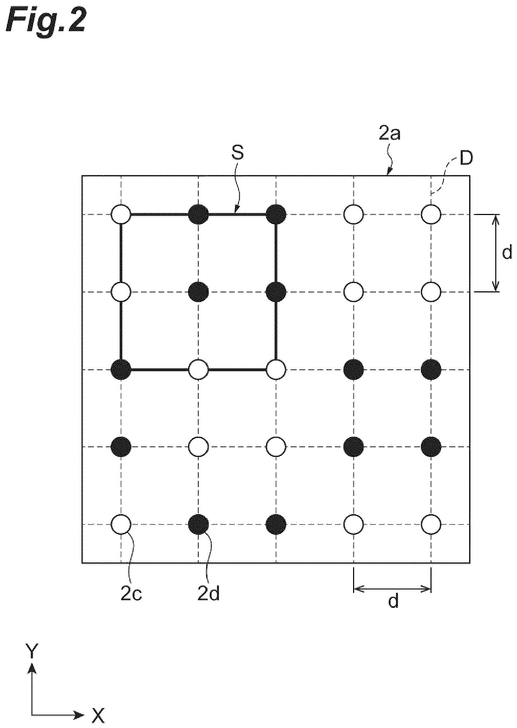

Here, an arrangement of light irradiation portions (light input portions) 2c and light receiving portions 2d on the contact surface 2a of the probe 2 will be described in detail. FIG. 2 is a plan view schematically illustrating the arrangement of the light irradiation portions 2c and the light receiving portions 2d. It should be noted that the light irradiation portions 2c are portions that irradiate the measurement target part B with light, and the plurality of light irradiation portions 2c are provided to correspond to the plurality of respective irradiation optical fibers 11a. Each of the light irradiation portions 2c is optically coupled to one end of a corresponding irradiation optical fiber 11a or is configured as one end of the corresponding irradiation optical fiber 11a. The light receiving portions 2d are portions that receive the diffused reflected light Lb emitted from the measurement target part B, and the plurality of light receiving portions 2d are provided to correspond to the plurality of respective detection optical fibers 11b. Each of the light receiving portions 2d is optically coupled to one end of a corresponding detection optical fiber 11b or configured as one end of the corresponding detection optical fiber 11b.

As illustrated in FIG. 2, a virtual square lattice D configured of a plurality of mutually parallel lines extending in an X direction (a first direction) and aligned in a Y direction (a second direction) and a plurality of mutually parallel lines extending in the Y direction and aligned in the X direction is set on the contact surface 2a. The X direction and the Y direction are orthogonal to each other. A lattice interval of this square lattice D is d. For example, d is 1 cm. This square lattice D includes a plurality of lattice points aligned at substantially equal intervals d in the X direction and the Y direction. It should be noted that saturation is likely to occur at the time of light detection when the interval d is small, and resolution becomes degraded when the interval d is great. Therefore, the interval d can be set to be equal to or greater than 0.5 cm and equal to or smaller than 1 cm.

The plurality of light irradiation portions 2c are arranged at some of the plurality of lattice points and the plurality of light receiving portions 2d are arranged at other lattice points among the plurality of lattice points. As an example, lattice points in five rows in the X direction and five columns in the Y direction (a total of 25 lattice points) are shown in FIG. 2, the light irradiation portion 2c are provided at 13 lattice points among the 25 lattice points, and the light receiving portions 2d are provided at the other 12 lattice points. A center position of each light irradiation portion 2c matches a position of the corresponding lattice point, and a center position of each light receiving portion 2d matches a position of the corresponding lattice point.

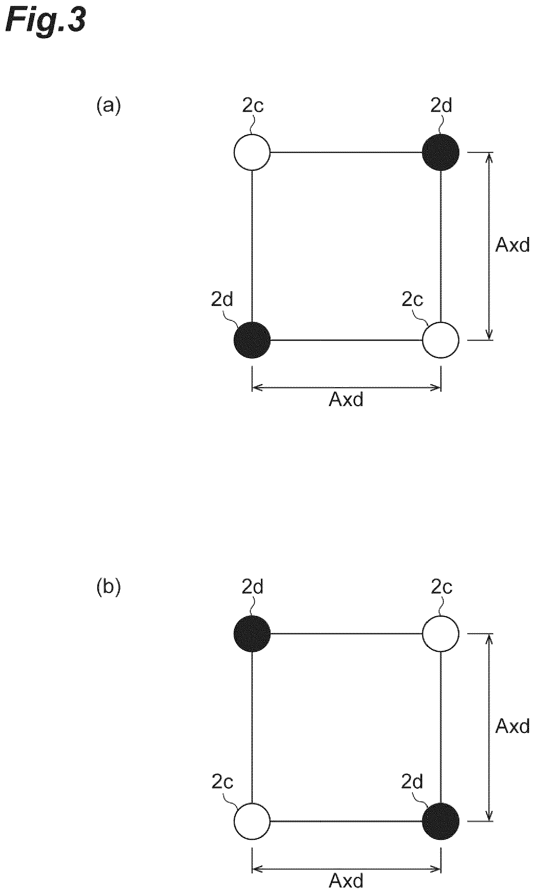

The light irradiation portions 2c and the light receiving portions 2d are arranged on the basis of a predetermined rule. That is, the light receiving portions 2d are necessarily arranged at each lattice point separated by a distance A×d (A is an integer equal to or greater than 2 and A is 2 in FIG. 2) in the X direction and each lattice point separated by the distance A×d in the Y direction from the lattice point at which the light irradiation portion 2c is arranged. Further, the light irradiation portion 2c is necessarily arranged at each lattice point separated by the distance A×d in the X direction and each lattice point separated by the distance A×d in the Y direction from each lattice point at which the light receiving portion 2d is arranged. In other words, in a square of which a length of a side is (A×d), the light irradiation portion 2c is arranged at two corners on one of diagonal lines, and the light receiving portion 2d is arranged at two corners on the other of the diagonal lines, as illustrated in FIGS. 3(a) and 3(b). By setting such an arrangement as a basic pattern and combining a plurality of basic patterns, the light irradiation portions 2c and the light receiving portions 2d are arranged so that the light irradiation portions 2c and the light receiving portions 2d are located on the lattice points of the square lattice D. It should be noted that a square S indicating one basic pattern is shown in FIG. 2.

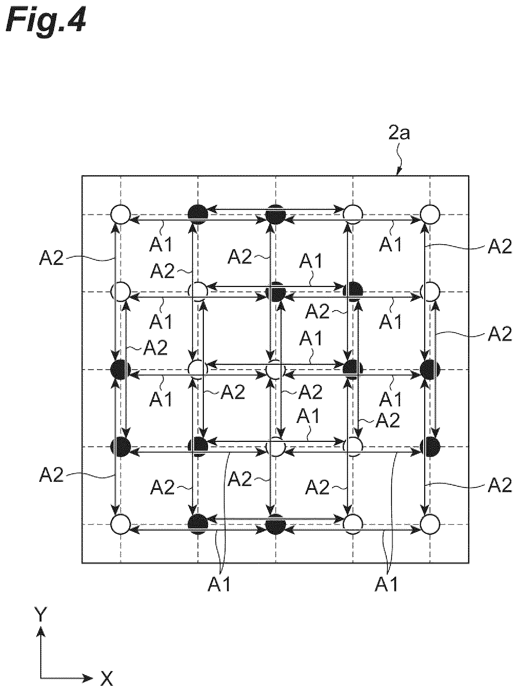

The internal information calculation unit 82 calculates internal information of the measurement target part B on the basis of a result of detection of the diffused reflected light Lb obtained for each of combinations of the light irradiation portions 2c and the light receiving portions 2d. Specifically, as illustrated in FIG. 4, the internal information calculation unit 82 calculates the internal information on the basis of a detection result of the diffused reflected light Lb obtained by a plurality of combinations (arrows A1 in FIG. 4) of the light irradiation portions 2c and the light receiving portions 2d separated by the distance A×d in the X direction and a plurality of combinations (arrows A2 in FIG. 4) of the light irradiation portion 2c and the light receiving portion 2d separated by a distance A×d in the Y direction. Therefore, in the present embodiment, a distance between irradiation and light reception in a set of the light irradiation portions 2c and the light receiving portions 2d that is used for detection of the diffused reflected light Lb is constant as A×d.

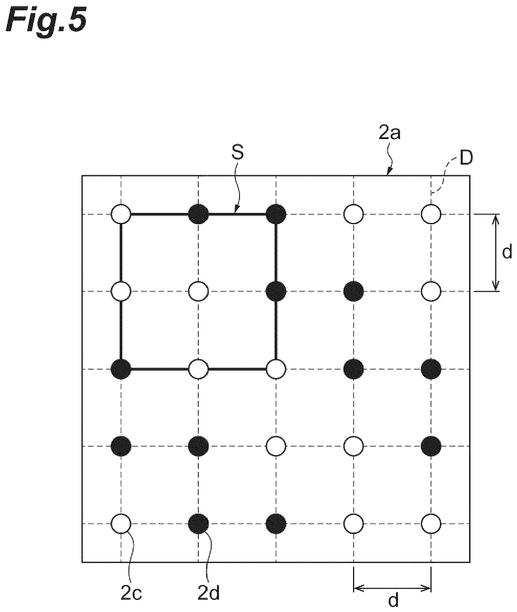

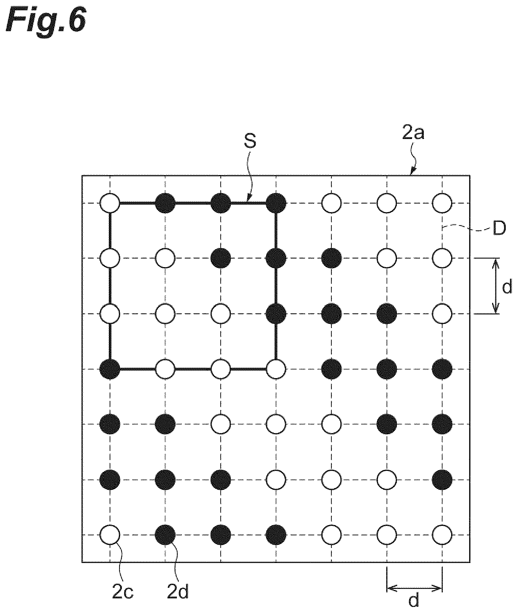

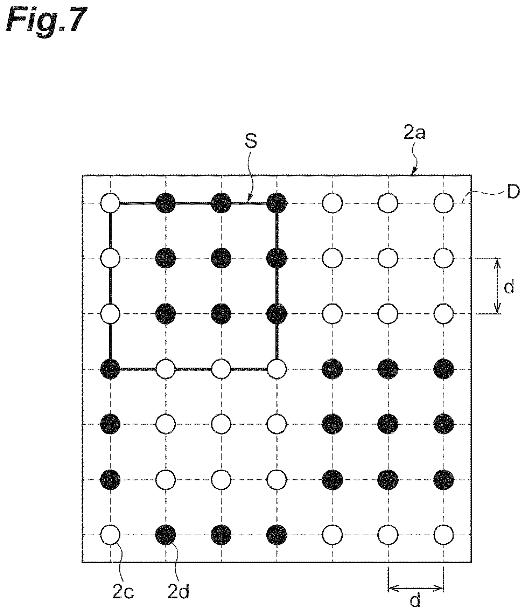

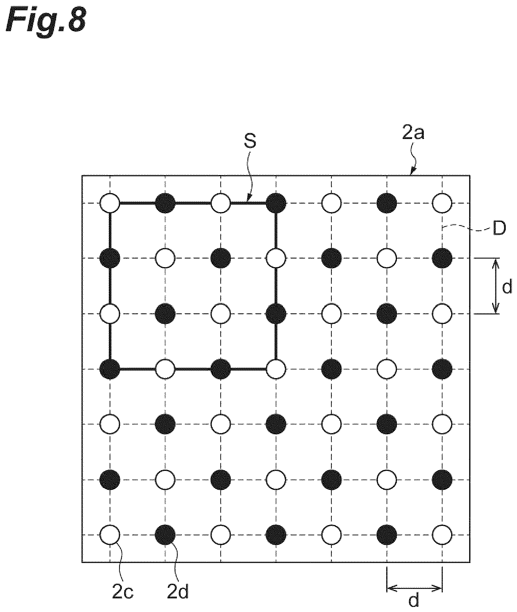

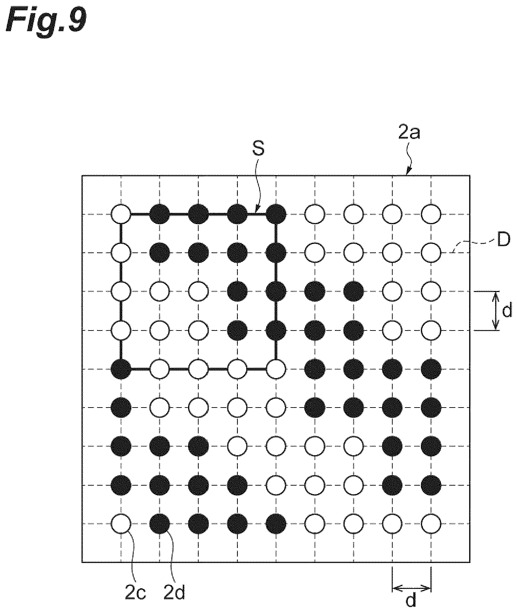

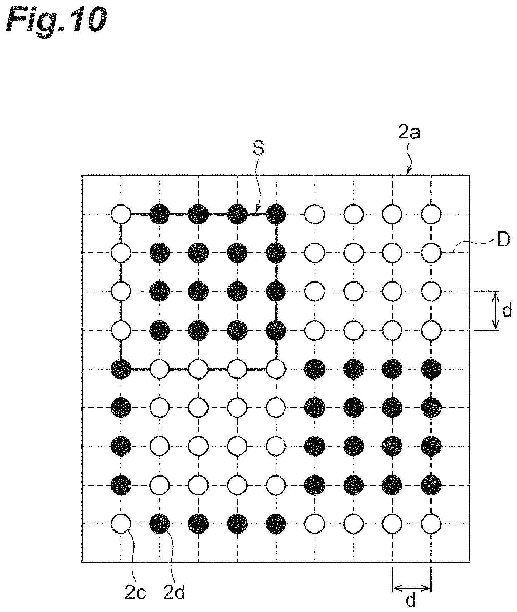

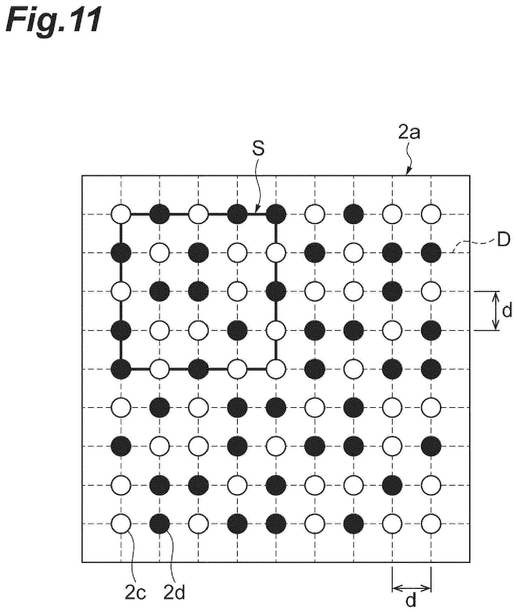

It should be noted that the arrangement of the light irradiation portions 2c and the light receiving portions 2d may be in accordance with a predetermined rule and is not limited to the arrangement illustrated in FIG. 2. FIG. 5 is a plan view schematically illustrating another example of the arrangement of the light irradiation portions 2c and the light receiving portions 2d. The example illustrated in FIG. 5 is a case in which constant A = 2 as in the case of FIG. 2 and the light irradiation portions 2c and the light receiving portions 2d are arranged on the basis of the same rule as in FIG. 2. However, the arrangement of the light irradiation portions 2c and the light receiving portions 2d is different from that in FIG. 2. Further, examples illustrated in FIGS. 6 to 8 correspond to a case in which constant A = 3, and the light irradiation portions 2c and the light receiving portions 2d are arranged on the basis of the same rule as in FIG. 2. In the examples illustrated in FIGS. 6 to 8, the distance between irradiation and light reception of the light irradiation portions 2c and the light receiving portion 2d is 3×d. When the distance between irradiation and light reception is made equal to that in FIG. 2, a length of the lattice interval d can be set to 2/3 of the lattice interval d in FIG. 2. Further, Examples illustrated in FIGS. 9 to 11 correspond to a case in which constant A = 4, and the light irradiation portions 2c and the light receiving portions 2d are arranged on the basis of the same rule as in FIG. 2. In the examples illustrated in FIGS. 9 to 11, the distance between irradiation and light reception of the light irradiation portions 2c and the light receiving portions 2d is 4×d. When the distance between irradiation and light reception is made equal to that in FIG. 2, the length of the lattice interval d can be set to 1/2 of the lattice interval d in FIG. 2.

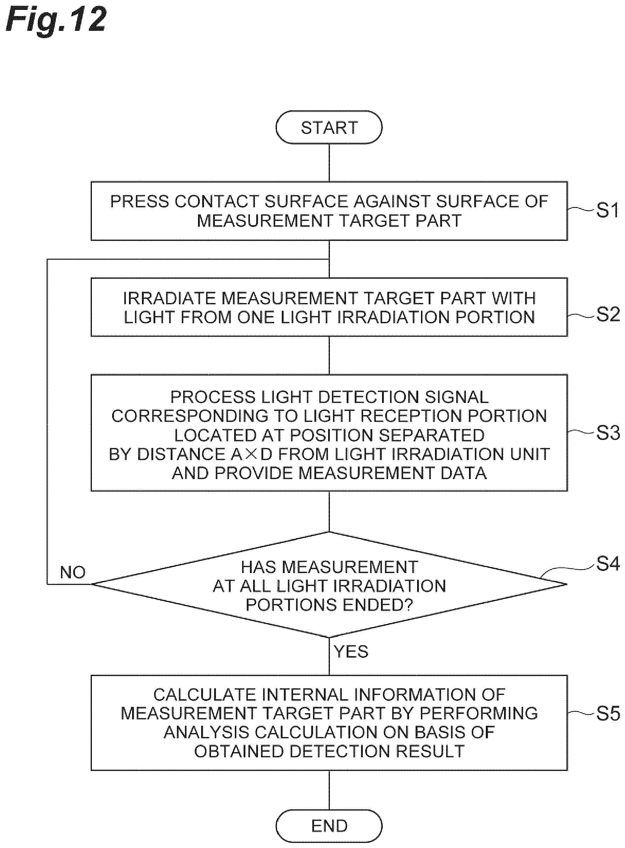

FIG. 12 is a flowchart illustrating a biological measurement method in the biological measurement device 1A. As illustrated in FIG. 12, in the biological measurement method of the embodiment, the contact surface 2a is first pressed against the surface Ba of the measurement target part B (arrangement step S1). One light irradiation portion 2c irradiates the measurement target part B with the light La (light input step S2). The light La propagates through the measurement target part B and is output as diffused reflected light Lb to the plurality of light receiving portions 2d. The diffused reflected light Lb output to each light receiving portion 2d is detected by the photodetector 5 corresponding to the light receiving portion 2d. Each photodetector 5 outputs the light detection signal Sd to the signal processing unit 7. The signal processing unit 7 processes the light detection signal Sd corresponding to the light receiving portion 2d located at a position separated by the distance A×d (A is an integer equal to or greater than 2) from the light irradiation portion 2c among the light detection signals Sd output from the respective photodetectors 5, and provides the measurement data Da as a detection result to the calculation processing unit 8 (detection step S3). When the measurement in the light irradiation portion 2c is completed, measurement in the next light irradiation portion 2c is performed. These operations are repeated until the measurement at all set light irradiation portions 2c ends (step S4). It should be noted that the signal processing unit 7 may not receive the light detection signal Sd corresponding to a light receiving portion 2d that is not in the relationship described above or may not process the light detection signal Sd.

The internal information calculation unit 82 calculates internal information of the measurement target part B by performing analysis calculation such as the TRS or the PMS on the basis of the obtained detection result (calculation step S5). Therefore, the internal information calculation unit 82 can calculate the internal information of the measurement target part B on the basis of the detection result corresponding to the combination in which an interval between the light irradiation portion 2c and the light receiving portion 2d becomes the distance A×d.

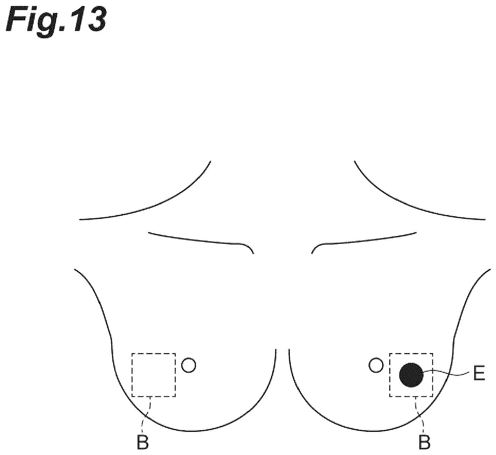

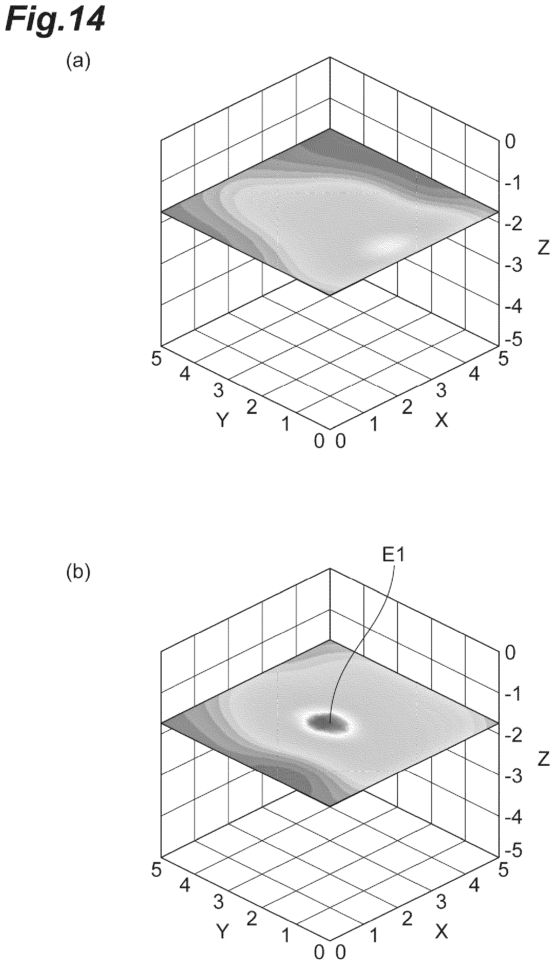

The internal information calculation unit 82 obtains an image (a CT image) of a three-dimensional distribution of an optical coefficient or concentrations of various light absorption substances in a range of depth from 0 cm to, for example, 3 to 4 cm in a region of the measurement target part B (for example, a region of 5 cm×5 cm) covered by the contact surface 2a of the probe 2. Here, an example in which the light absorption substance concentration calculated by the internal information calculation unit 82 is actually formed as an image will be described. FIG. 13 is a front view illustrating left and right breasts of a breast cancer patient including a measurement target part B which is a measurement target, and a tumor E having a diameter of about 20 mm is present in the left breast. FIGS. 14(a) and 14(b) illustrate a result of measuring the measurement target part B illustrated in FIG. 13 and forming a CT image. It should be noted that, for ease of understanding, cross sections at a certain position (Z = 17 mm) in a depth direction of a three-dimensional CT image are illustrated in FIGS. 14(a) and 14(b). FIG. 14(a) illustrates a CT image regarding the right breast in which a tumor E is not generated, and FIG. 14(b) illustrates a CT image regarding the left breast including a tumor E. It can be seen from a comparison of the CT images with each other that, in FIG. 14(b), an image E1 regarding the tumor E, which is not illustrated in FIG. 14(a), is clearly included.

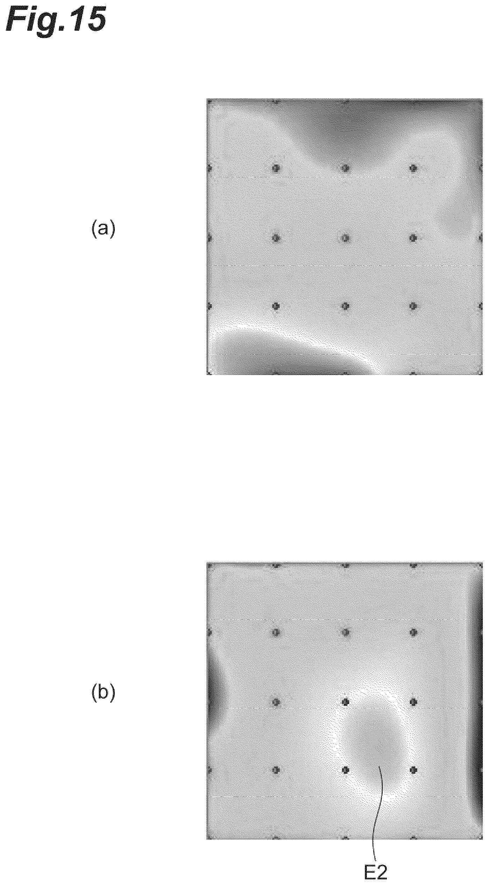

Further, in addition to the three-dimensional CT image described above, the internal information calculation unit 82 can calculate an optical coefficient or concentrations of various light absorption substances in a lattice form at intervals (for example, intervals of 1 cm) equal to the lattice interval d using, for example, TRS of one channel and map an obtained value as a two-dimensional image. That is, it is possible to obtain both a three-dimensional image (a CT image) and a two-dimensional image (a mapping image) in one measurement. FIGS. 15(a) and 15(b) illustrate a two-dimensional mapping image obtained by actually measuring the measurement target part B illustrated in FIG. 13. FIG. 15(a) illustrates a two-dimensional mapping image regarding the right breast in which the tumor E is not generated, and FIG. 15(b) illustrate a two-dimensional mapping image regarding the left breast including the tumor E. It can be seen from a comparison of the two-dimensional mapping images with each other that an image E2 regarding the tumor E, which is not present in FIG. 15(a), is clearly included in FIG. 15(b).

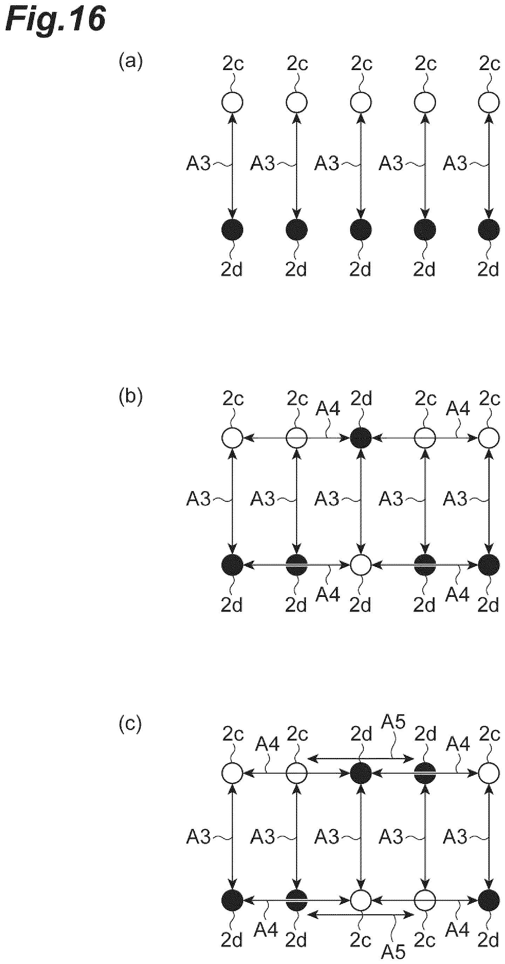

Effects that are obtained by the biological measurement device 1A according to the embodiment described above will be described. FIG. 16(a) to 16(c) are diagrams illustrating a process of the embodiment. First, as illustrated in FIG. 16(a), it is assumed that pairs of the light irradiation portion 2c and the light receiving portion 2d having a predetermined distance between irradiation and light reception are equally aligned in a direction orthogonal to an alignment direction of the pairs. In this case, only measurement data in the alignment direction (a vertical direction in FIG. 16) of the light irradiation portion 2c and the light receiving portion 2d can be obtained (arrows A3 in FIG. 16(a)), and measurement data in a horizontal direction is insufficient. Then, as illustrated in FIG. 16(b), the light irradiation portion 2c and the light receiving portion 2d of the pair located at a center are interchanged. In this case, measurement data in a horizontal direction is obtained (arrows A4 in FIG. 16(b)), in addition to measurement data in a vertical direction illustrated in FIG. 16(a). Further, the light irradiation portion 2c and the light receiving portion 2d in the pair adjacent to the pair located at the center are interchanged. In this case, measurement data in the horizontal direction further increases (arrows A5 in FIG. 16(c)), in addition to the measurement data illustrated in FIG. 16(b). As a result of such an examination, it has been concluded that it is preferable for the light irradiation portions 2c and the light receiving portions 2d to be arranged according to the combination of the basic patterns illustrated in FIGS. 3(a) and 3(b) in order to acquire the measurement data in the horizontal direction (the X direction in FIG. 2) and the measurement data in the vertical direction (the Y direction in FIG. 2) as much as possible as the measurement data at the predetermined distance between irradiation and light receiving. It has been found that a density of measurement data becomes uniform and measurement sensitivity becomes substantially uniform by arranging the light irradiation portions 2c and the light receiving portions 2d to be aligned in a square lattice form having equal intervals in the vertical and horizontal directions.

That is, a density of the combinations of the light irradiation portions 2c and the light receiving portions 2d becomes uniform within the contact surface 2a by arranging a plurality of light irradiation portions 2c and a plurality of light receiving portions 2d on the contact surface 2a as illustrated in FIGS. 2 and 5 to 11 and setting the combinations of the light irradiation portions 2c and light receiving portions 2d for detecting the diffused reflected light Lb as described above. Therefore, it is possible to improve the uniformity of measurement sensitivity and obtain a clearer image.

It should be noted that a configuration in which the combinations of the light irradiation portions and the light receiving portions are made uniform can also be realized by using a coaxial fiber in which both light irradiation and light reception can be performed at one end. However, in this case, coaxial fibers are required by the number of lattice points (25 lattice points in FIG. 2), and the number of light source devices and the number of photodetectors are also required by the number of lattice points. On the other hand, in the embodiment, since measurement with the same sensitivity can be realized with a smaller number of light irradiation portions 2c and light receiving portions 2d, it is possible to avoid complication of the device configuration and greatly suppress manufacturing cost.

Further, in the embodiment, the constant A is the integer equal to or greater than 2. However, in a case in which the constant A is 1 (that is, when the lattice interval d is equal to the distance between irradiation and light reception), an intensity of light incident on the light receiving portion 2d may be too high for example, when a concentration of a metabolite inside a body is obtained using the TRS, and there is concern that a dynamic range is deteriorated. When the lattice interval d is increased in order to solve this problem, the number of pieces of measurement data obtained decreases, and therefore, a reconstructed image deteriorates. On the other hand, in the embodiment, since the constant A is equal to or greater than 2, it is possible to acquire a sufficient number of pieces of measurement data while suppressing an intensity of light incident on the light receiving portions 2d. Therefore, it is possible to improve measurement accuracy and acquire a clearer image.

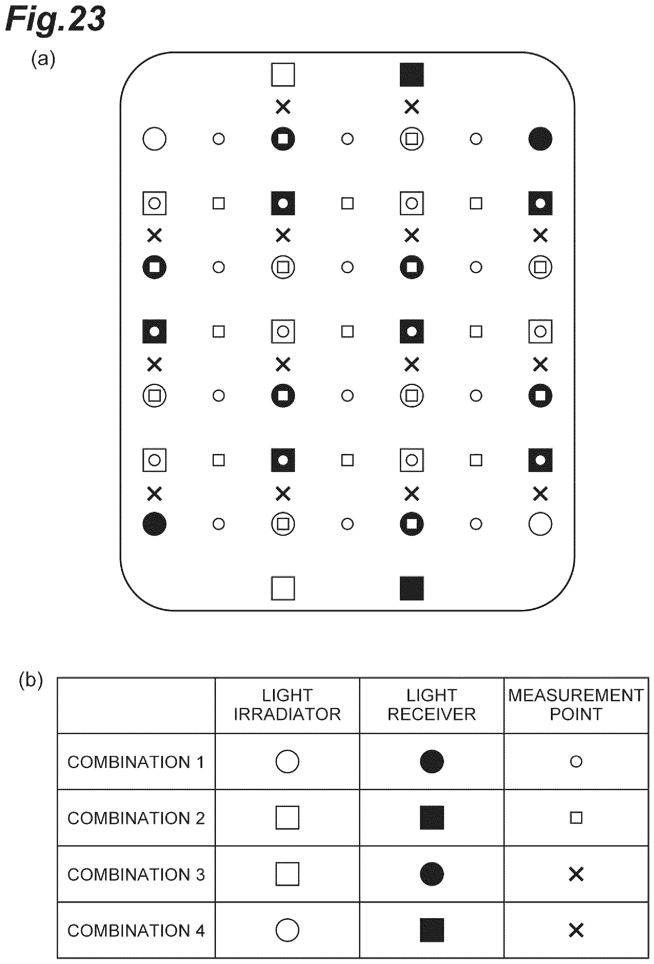

Further, FIGS. 23(a) and 23(b) are diagrams illustrating an arrangement of light irradiators and light receivers described in Patent Literature 1, in which combinations of the light irradiators and the light receivers, and measurement points in each combination are illustrated. In the arrangement illustrated in FIGS. 23(a) and 23(b), light irradiators and light receivers are arranged so that measurement points are aligned vertically and horizontally at predetermined intervals. However, at any of the measurement points, an alignment direction of the light irradiator and the light receiver is only one of the vertical direction and a horizontal direction. In order to further improve the accuracy of the measurement, it is ideal to cause light to propagate through one measurement point from as many directions as possible. However, in order to improve uniformity of measurement sensitivity, it is possible to cause light to propagate from at least two directions and perform measurement. Further, in the arrangement of the light irradiators and the light receivers illustrated in FIG. 23(a), since the distance between irradiation and light reception is different between the vertical direction and the horizontal direction, measurement data at a uniform distance between irradiation and light reception cannot be acquired, and the uniformity of the measurement sensitivity is degraded. The biological measurement device 1A of the embodiment solves the problem of the device described in Patent Literature 1 as described above and obtains a clearer image by improving the uniformity of the measurement sensitivity.

Further, the plurality of light irradiation portions 2c may sequentially input light to the measurement target part B, as in the embodiment. Accordingly, it is possible to avoid interference between light beams that are input from the plurality of light irradiation portions 2c and improve S/N.

Further, the integer A may be 2, as in the embodiment. Accordingly, it is possible to increase the number of combinations of the plurality of light irradiation portions 2c and the plurality of light receiving portions 2d. Therefore, the uniformity of the measurement sensitivity and the resolution are further improved and a clearer image can be obtained.

Further, the lattice interval d may be equal to or greater than 0.5 cm and less than or equal to 1 cm, as in the present embodiment. In this case, it is possible to improve resolution while suppressing saturation at the time of light detection.

Further, the calculation processing unit 8 (a calculation step) may create a two-dimensional image or a three-dimensional image on the basis of the internal information, as in the embodiment. According to the embodiment, since the density of the combinations of the light irradiation portions 2c and the light receiving portions 2d becomes uniform as described above, it is possible to create a clear image.

Here, examples and comparative examples for verification of the above effects will be described. The present inventor prepared a probe 2 having the arrangement of the light irradiation portions 2c and the light receiving portions 2d illustrated in FIG. 2 and performed a verification experiment shown below. It should be noted that the lattice interval d was 10 mm and the constant A was 2.

First, as an example, a reconstructed image regarding absorption coefficient was obtained by measuring a solid phantom including an absorber therein using only a detection result of the diffused reflected light Lb (that is, measurement data in which the distance between irradiation and light reception is 20 mm) obtained by a plurality of combinations (the arrows A1 in FIG. 4) of the light irradiation portions 2c and the light receiving portions 2d separated by a distance of 20 mm in the X direction and a plurality of combinations (the arrows A2 in FIG. 4) of the light irradiation portions 2c and the light receiving portions 2d separated by a distance of 20 mm in the Y direction.

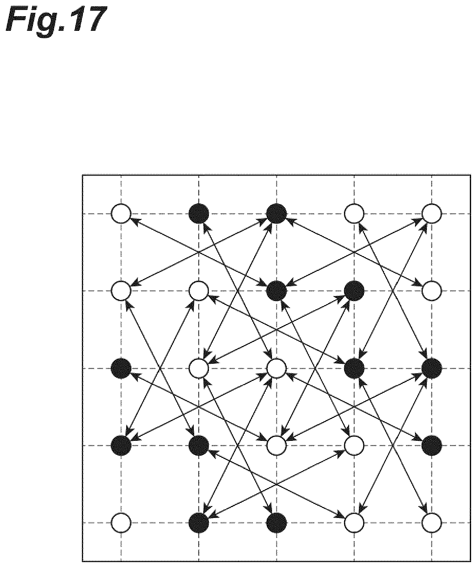

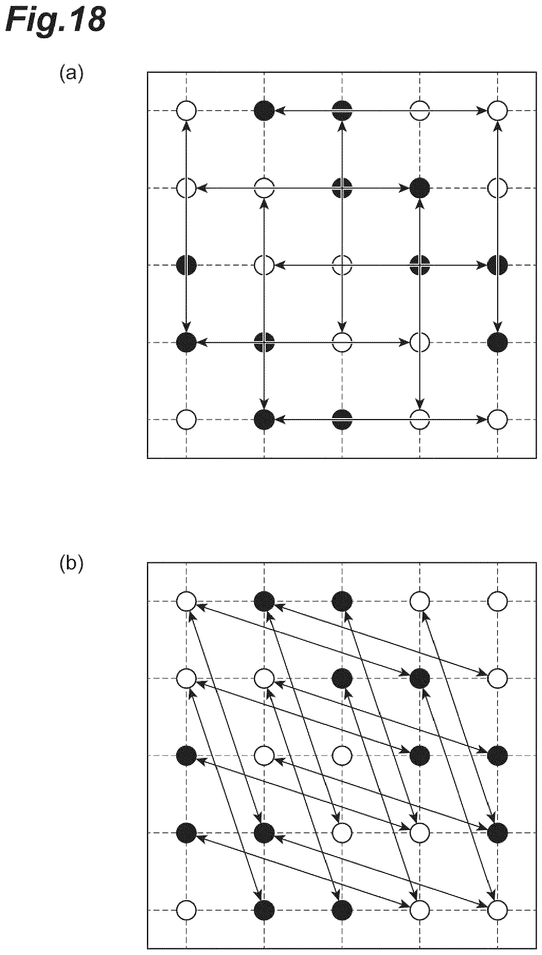

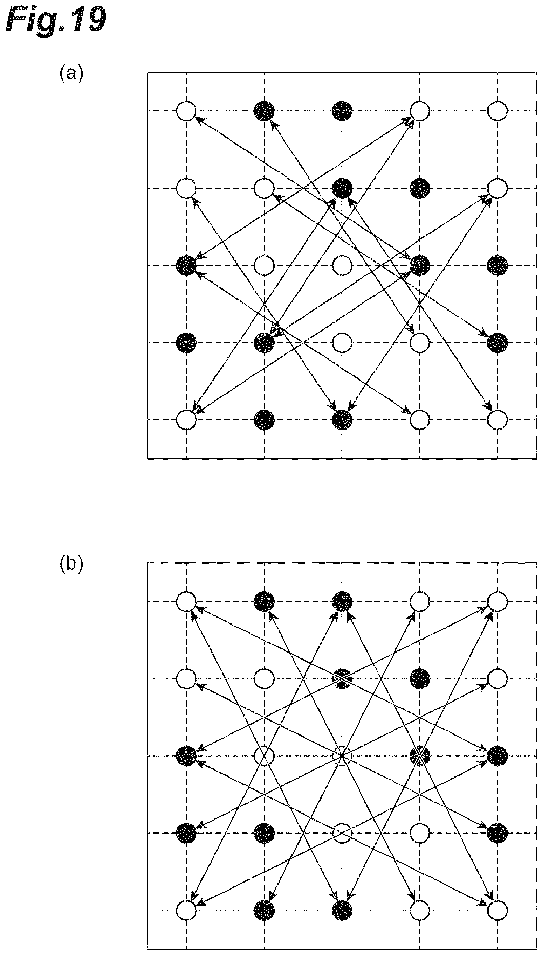

Next, in a comparative example, combinations of light irradiation portions 2c and light receiving portions 2d were changed to be nonuniform, and a solid phantom was measured to obtain a reconstructed image regarding an absorption coefficient. FIGS. 17, 18(a), 18(b), 19(a), and 19(b) are diagrams illustrating combinations of the light irradiation portions 2c and the light receiving portions 2d in the comparative example, and a double arrow in each diagram indicate a combination of the light irradiation portion 2c and the light receiving portion 2d. In these diagrams, distances between irradiation and light reception are 2.24 cm, 3 cm, 3.16 cm, 3.61 cm, and 4.47 cm, respectively. In the comparative example, one reconstructed image was created by using measurement data of the distances between irradiation and light reception together.

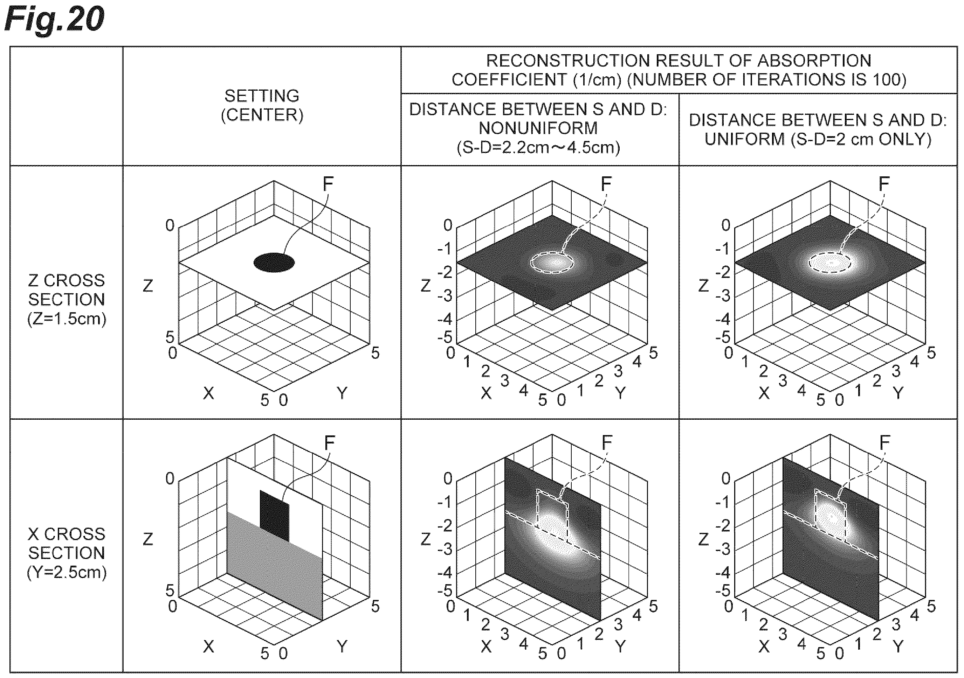

FIG. 20 is a diagram illustrating a position and a shape of an absorber F inside a solid phantom (two upper and lower diagrams at a left end), CT images (two upper and lower diagrams at a center) indicating a three-dimensional distribution of an absorption coefficient inside the solid phantom acquired in the comparative example, and CT images (two upper and lower diagrams on a right end) indicating a three-dimensional distribution of the absorption coefficient inside the solid phantom acquired in the example. It should be noted that, for ease of understanding, the three upper diagrams show cross sections at a certain position in a depth direction (Z = 1.5 cm), and the three lower diagrams show cross sections at a certain position in a Y direction (Y = 2.5 cm).

Referring to FIG. 20, it can be seen that in the comparative example (see the two diagrams at the center), a pixel value is small (that is, sensitivity is low) and the position and the size of the absorber F inside the solid phantom cannot be accurately measured. On the other hand, in the example, the pixel value is great (that is, the sensitivity is high) in spite of the number of pieces of measurement data being half or smaller than the number in the comparative example, and the position and the size of the absorber F inside the solid phantom can be accurately measured.

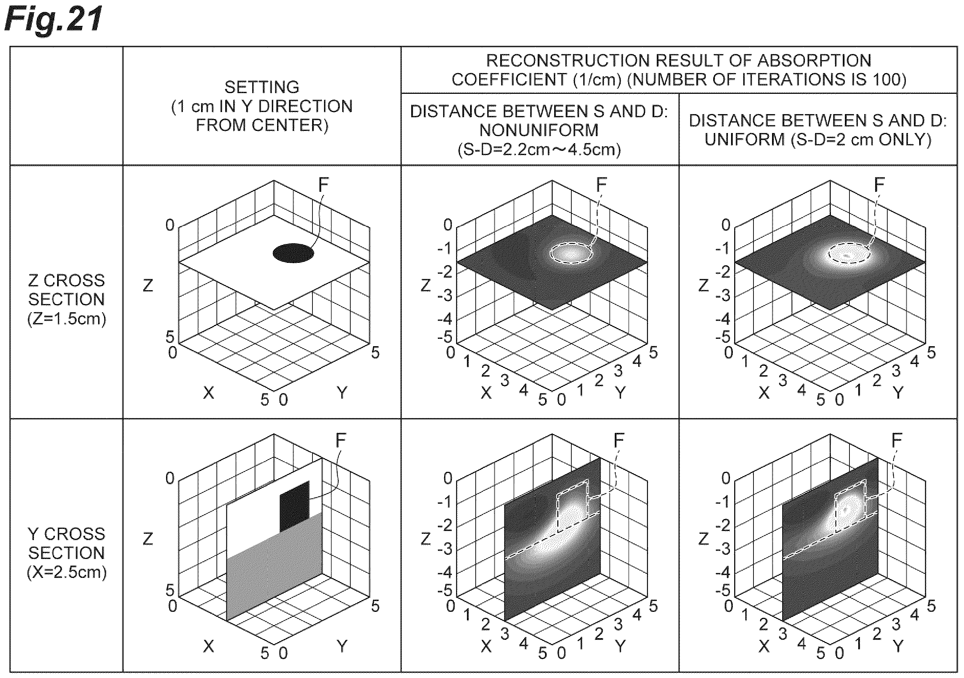

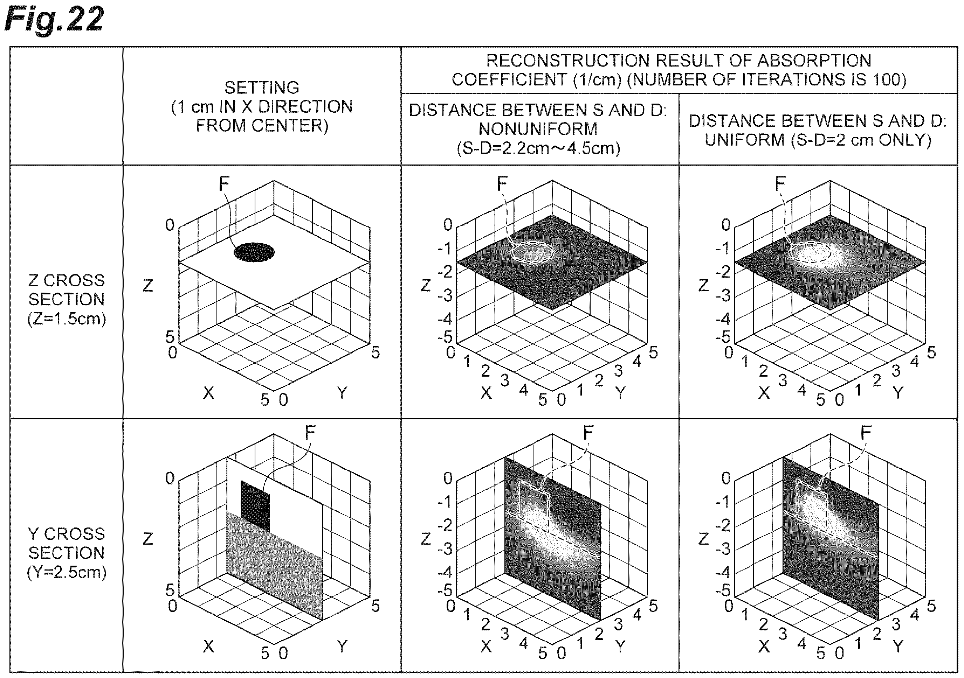

It should be noted that, in order to confirm uniformity in a lateral direction (a direction within an XY plane), the example was compared with the comparative example when the position of the absorber F was shifted by 1 cm in the Y direction from the position illustrated in FIG. 20 (FIG. 21) and when the position of the absorber F was shifted by 1 cm in the X direction (FIG. 22). As a result, it was confirmed that, in the example, the sensitivity was higher and the uniformity in the lateral direction was improved as compared with the comparative example.

The biological measurement device and the biological measurement method according to the aspect of the present invention are not limited to the above-described embodiments, and various other modifications are possible. For example, the arrangement of the light irradiation portions and the light receiving portions is not limited to each of the forms illustrated in FIG. 2 and FIGS. 5 to 11, and can be various arrangements according to the rule described in the above embodiment. Further, the probe 2 may include the light source device 4 and the photodetector 5, the light source device 4 may be arranged in the light irradiation portion 2c, and the photodetector 5 may be arranged in the light receiving portion 2d.

Further, the biological measurement device is a biological measurement device that inputs light to a measurement target part of a subject and acquires internal information of the measurement target part by detecting measurement light propagating through the measurement target part, the biological measurement device including: a probe including a contact surface that includes a plurality of light input portions that input the light to the measurement target part and a plurality of light receiving portions that receive the measurement light output from the measurement target part, the contact surface being pressed against the measurement target part; and a calculation unit that calculates the internal information on the basis of a result of detecting the measurement light obtained for each combination of the light input portion and the light receiving portion, wherein the light input portions and the light receiving portions may be arranged at some lattice points among a plurality of lattice points included in the lattice having a lattice interval d in a first direction and a second direction orthogonal to each other, and a plurality of light receiving portions may be arranged at other lattice points among the plurality of lattice points, the light receiving portions may be arranged at each lattice point separated by a distance A×d (A is an integer equal to or greater than 2) in the first direction from each lattice point at which the light input portion is arranged and each lattice point separated by the distance A×d in the second direction from each lattice point at which the light input portion is arranged, the light input portions may be arranged at each lattice point separated by the distance A×d in the first direction from each lattice point at which the light receiving portion is arranged and each lattice point separated by the distance A×d in the second direction from each lattice point at which the light receiving portion is arranged, and the calculation unit may calculate the internal information on the basis of a detection result of the measurement light obtained by a plurality of combinations of the light input portions and the light receiving portions separated by the distance A×d in the first direction and a plurality of combinations of the light input portions and the light receiving portions separated by the distance A×d in the second direction.

### Industrial Applicability

It is possible to provide a probe type ecological measurement device and a biological measurement method capable of improving uniformity of measurement sensitivity.

### Reference Signs List

1A Biological measurement device
2 Probe
2a Contact surface
2c Light irradiation portion (light input portion)
2d Light receiving portion
4 Light source device
5 Photodetector
6 Synchronization signal generation unit
7 Signal processing unit
8 Calculation processing unit (calculation unit)
11a Irradiation optical fiber
11b Detection optical fiber
81 Measurement data storage unit
82 Internal information calculation unit
B Measurement target part
Ba Surface
D Square lattice
Da Measurement data
E Tumor
F Absorber
La Light
Lb Diffused reflected light (measurement light)
Sd Light detection signal
St Trigger signal

## Claims

1. A biological measurement device that inputs light to a measurement target part of a subject and acquires internal information of the measurement target part by detecting measurement light propagating through the measurement target part, the biological measurement device comprising:
a probe including a contact surface that includes a plurality of light input portions that input the light to the measurement target part and a plurality of light receiving portions that receive the measurement light output from the measurement target part, the contact surface being pressed against the measurement target part; and
a calculation unit that calculates the internal information on the basis of a result of detecting the measurement light obtained for each combination of the light input portion and the light receiving portion,
wherein the plurality of light input portions and the plurality of light receiving portions constitute lattices having an interval d, along first direction and a second direction orthogonal to each other on the contact surface,
at least some of the plurality of light receiving portions are located at a position separated by a distance A×d (A is an integer equal to or greater than 2) in the first direction and the second direction from the light input portion,
at least some of the plurality of light input portions are located at a position separated by the distance A×d in the first direction and the second direction from the light receiving portion, and
the combination is a combination in which an interval between the light input portion and the light receiving portion is the distance A×d.

2. The biological measurement device according to claim 1, wherein the plurality of light input portions sequentially input the light to the measurement target part.

3. The biological measurement device according to claim 1 or 2, wherein the integer A is 2.

4. The biological measurement device according to any one of claims 1 to 3, wherein the lattice interval d is equal to or greater than 0.5 cm and less than or equal to 1 cm.

5. The biological measurement device according to any one of claims 1 to 4, wherein the calculation unit creates a two-dimensional image or a three-dimensional image on the basis of the internal information.

6. A biological measurement method for inputting light to a measurement target part of a subject and acquiring internal information of the measurement target part by detecting measurement light propagating through the measurement target part, the biological measurement method comprising steps of:
disposing a probe including a contact surface that includes a plurality of light input portions and a plurality of light receiving portions, at the measurement target part;
inputting the light from one of the plurality of light input portions to the measurement target part;
detecting the measurement light received by the light receiving portion located at a position separated by a distance A×d (A is an integer equal to or greater than 2) from the light input portion that has input the light among the plurality of light receiving portions when an interval between lattices configured of the plurality of light input portions and the plurality of light receiving portions on the contact surface is d, and outputting a detection result; and
calculating the internal information on the basis of the detection result.
